# EUROPEAN PATENT APPLICATION

(11) **EP 4 442 813 A1**
(43) Date of publication of application: **09.10.2024**
(21) Application number: 22901635.7
(22) Date of filing: 17.11.2022
(51) Int. Cl.: C12N 5/0783, C12N 15/113, C07K 14/705, A61K 35/17, A61P 35/00

(54) **IMMUNE CELLS WITH ENHANCED EFFICACY**

(30) Priority: 02.12.2021 KR 20210171191
(71) Applicant: Korea Research Institute of Chemical Technology, Daejeon 34114 (KR); Abtironbio Co., Ltd., Hanam-si, Gyeonggi-do 12918 (KR)
(72) Inventor: PARK, Young Kwang, Namyangju-si, Gyeonggi-do 12278 (KR); PARK, Chi Hoon, Daejeon 34114 (KR); KIM, Yeong Rin, Daejeon 34114 (KR); CHOI, Sang Un, Daejeon 34114 (KR); LEE, Heung Kyoung, Daejeon 34114 (KR)
(74) Representative: Maiwald GmbH
(86) International application number: PCT/KR2022/018215
(87) International publication number: WO 2023/101277

(57) **Abstract**

The present invention relates to a composition for improving immune cell activity comprising a TET2 expression inhibitor, and a method for preparing immune cells with improved activity. In addition, the present invention relates to a recombinant vector comprising a nucleic acid encoding a TET2 inhibitor, immune cells with improved activity transduced with the recombinant vector, and a pharmaceutical composition for treating cancer comprising the immune cells.

## Description

### TECHNICAL FIELD

The present invention relates to a composition for improving immune cell activity, a method for preparing immune cells with improved activity, immune cells with improved activity, and a use thereof.

### BACKGROUND ART

Cell therapeutic agents are pharmaceutical drugs that induce therapeutic efficacy such as regeneration using living cells to restore damaged or diseased cells, tissues, and individuals, and among them, immunomodulatory cell therapeutic agents are pharmaceutical drugs used to treat diseases by controlling an immune response in the body using immune cells such as dendritic cells, natural killer cells (NK cells), T cells, and the like.

The immunomodulatory cell therapeutic agent is used by activating various immune cells such as peripheral blood mononuclear cells (PBMCs), T cells, and NK cells isolated from a patient with various antibodies and cytokines, proliferating these immune cells in vitro, and then injecting the proliferated immune cells back into the patient or injecting immune cells into which a gene such as a T-cell receptor (TCR) or a chimeric antigen receptor (CAR) is introduced back into the patient.

A CAR is an artificial chimeric protein in which a single chain antibody that recognizes a cell surface antigen on a cancer cell is fused with a signal transduction domain that induces activation of T cells. By introducing a gene encoding a CAR into normal peripheral blood T cells (peripheral blood T lymphocytes) that do not have tumor reactivity, it is possible to prepare CAR-expressing T cells (hereinafter, referred to as "CAR-T cells") that express a CAR in large quantities. These CAR-T cells have tumor reactivity, which makes it possible to induce cancer cell injury without relying on interaction with the major histocompatibility gene complex (MHC).

A CAR-T cell therapeutic agent has attracted attention because it exerts dramatic effects in clinical trials, particularly for a hematologic tumor. That is, in the case of CAR-T cell treatment using an antibody that recognizes CD19, which is a B-lymphocyte hematologic tumor antigen, in early clinical trials targeting patients with acute lymphocytic leukemia who had not responded to all existing treatments, 90% of the patients (27 out of 30) achieved complete remission within one month, and the 6-month overall survival rate reached 78%, showing remarkable treatment effects. Based on these results, two types of CD19 CAR-T cell therapeutic agents were successfully commercialized under FDA approval at the end of 2017.

Currently, successful cases of CAR-T cell treatment are limited to CD19 positive acute leukemia, and in the case of solid tumors, the treatment efficiency is reported to be low. As an example, in a hematologic tumor where CAR-T cells are exposed to abundant amounts of malignant B cells in lymph nodes or during the treatment of highly immunogenic tumors such as melanoma, a strong activation profile and a strong cytotoxic effect are caused, and in contrast, during CAR-T therapy for solid tumors, weakly activated T cells generated from limited exposure to tumor antigens cause unstable immune responses, lethargic clonal expansion, and premature clonal contraction.

Various methods to overcome these problems of clonal contraction and weak T cell activation have been adopted with moderate success. For example, second-generation CAR-T cells in which a CD28 signaling molecule was added to an intracellular portion of a CAR were prepared in order to overcome clonal contraction, promote rapid proliferation, and to overcome cytokine deficiency. However, the second-generation CAR-T cells were less effective in treating solid tumors. Accordingly, further modifications were performed to prepare a "third-generation" CAR with added costimulatory molecules such as 41BB and/or OX40. In addition, patients are routinely treated with IL-2 therapy in order to keep the transferred T cells alive and functional, resulting in production of cytokines uncontrolled by therapeutic T cells. Although these methods have been able to enhance T cell activation to a certain extent in the treatment of solid tumors, there is still a need to further improve the function of T cells by completely overcoming clonal contraction and promoting rapid proliferation and activation of T cells.

An RNA interference (RNAi) mechanism in which siRNA, shRNA, and the like inhibit gene expression within cells has high potential as a therapeutic agent in terms of controlling genes related to cancer and infectious diseases, and gene therapy using the RNAi phenomenon is receiving new attention in the treatment of incurable cancer diseases. Since RNAi has high sequence specificity, an RNAi system may specifically knock down one type of transcript and does not affect other mRNAs having similar sequences. These characteristics allow the RNAi mechanism to have potential and value in gene expression inhibition, gene function research, and drug target validation. In addition, the RNAi mechanism may be used to treat (1) a disease caused by over-expression or mis-expression of genes; and (2) a related disease including a disease caused by a gene mutation.

### DISCLOSURE OF THE INVENTION

### TECHNICAL PROBLEM

An object of the present invention is to provide a composition for improving immune cell activity and a method for preparing immune cells with improved activity.

Another object of the present invention is to provide a recombinant vector for preparing immune cells with improved activity.

Still another object of the present invention is to provide immune cells with improved activity.

Still another object of the present invention is to provide a pharmaceutical composition for treating cancer containing immune cells with improved activity.

### TECHNICAL SOLUTION

In order to achieve the above objects, an aspect of the present invention provides a composition for improving immune cell activity containing a tet methylcytosine dioxygenase 2 (TET2) inhibitor.

Another aspect of the present invention provides a method for preparing immune cells with improved activity, the method including injecting a TET2 inhibitor into immune cells.

Still another aspect of the present invention provides a recombinant vector containing a nucleic acid encoding a TET2 inhibitor.

Still another aspect of the present invention provides immune cells with improved activity transduced with the recombinant vector.

Still another aspect of the present invention provides a pharmaceutical composition for treating cancer containing the immune cells with improved activity as an active ingredient.

### ADVANTAGEOUS EFFECTS

In the present invention, a target sequence and shRNA that effectively knock down TET2, which has a long cDNA of approximately 6,000 bp, were selected and used to prepare immune cells in which TET2 is knocked down. The immune cells in which TET2 is knocked down by shRNA of the present invention exhibit improved tumor treatment efficiency compared to immune cells in which TET2 is not knocked down or in which TET2 is knocked down by other types of shRNAs, and may thus be efficiently used in immune cell treatment for cancer treatment.

The effects of the present invention are not limited to the effects mentioned above, and other effects that are not mentioned may be obviously understood by those skilled in the art from the following description.

### BRIEF DESCRIPTION OF THE DRAWINGS

FIG. 1 illustrates the results of confirming knockdown efficiency of TET2 according to an shRNA reaction in 293T cells in which TET2 shRNA is introduced by Western blot.
A of FIG. 2 illustrates a schematic view of a recombinant vector containing a nucleic acid encoding a chimeric antigen receptor (CAR) and a nucleic acid encoding a TET2 inhibitor, and B of FIG. 2 illustrates CD19 CAR gene.
FIG. 3 illustrates the results of confirming whether a recombinant vector is introduced into T cells and express a CAR by Western blot.
FIG. 4 illustrates the results of measuring expression levels of PD-1 and Tim-3, which are immunosuppressive receptors, by FACS to confirm whether CAR-T cells in which TET2 is knocked down have improved activity compared to normal T cells and CAR-T cells.
FIG. 5 illustrates a schematic experimental protocol to confirm an effect of improving in vivo anticancer efficacy of CAR-T cells in which TET2 is knocked down.
FIG. 6 illustrates a degree of tumor proliferation in vivo through intensity of in vivo luminescence emitted by a tumor cell population using an imaging device (IVIS, PerkinElmer) after administering CAR-T cells in which TET2 is knocked down to immunodeficient mice.
FIG. 7 illustrates survival rates of the mice after administering CAR-T cells in which TET2 is knocked down to the immunodeficient mice.
FIG. 8 is a diagram obtained by confirming tumor cell suppressing activity after administering CAR-T cells to cells expressing CD19 or cells not expressing CD19 to confirm in vitro anticancer efficacy of CD19 CAR-T cells expressing sh-A or sh-I.

### BEST MODE FOR CARRYING OUT THE INVENTION

Hereinafter, the present invention will be described in detail.

An aspect of the present invention provides a composition for improving immune cell activity containing a tet methylcytosine dioxygenase 2 (TET2) inhibitor.

The improvement of immune cell activity may be one or more selected from the group consisting of survival rate improvement, proliferation rate improvement, and cytokine expression increase. Immune cells with improved activity may show significantly improved tumor treatment efficiency compared to normal immune cells.

In a specific embodiment of the present invention, it was confirmed that in CAR-T cells into which a TET2 inhibitor was introduced, expression of immunosuppressive receptors such as PD-1 and TIM-3 was reduced compared to CAR-T cells into which a TET2 inhibitor was not introduced, and therefore, the activity of immune cells was improved by TET2 inhibition.

The "tet methylcytosine dioxygenase (TET2)" is one of genes (TET1, TET2, and TET3) encoding a protein that converts 5-methylcytosine into 5-hydroxymethylcytosine, and is known to be involved in maintaining DNA methylation. DNA methylation/demethylation is dynamically regulated throughout hematopoietic differentiation, and TET proteins regulate a gene expression level through a balance of DNA methylation during hematopoiesis and immune cell activation and expansion. Sequence information of mRNA and protein of the TET2 gene may be confirmed in known gene databases. For example, the nucleic acid sequence of human TET2 may be confirmed in Genbank No. BC150180.1 and the like.

The "TET2 inhibitor" is used to refer to all agents that reduce the expression or activity of TET2, and may include all agents that reduce the activity of TET2 by reducing the expression level of TET2 at a transcription level, mRNA level, or translation level, or by interfering with the activity of TET2. The TET2 inhibitor may be used without limitation in its form, such as a compound, nucleic acid, peptide, or virus capable of targeting TET2 and inhibiting expression or activity thereof, or a vector containing the nucleic acid. For example, the TET2 inhibitor may be miRNA, siRNA, or shRNA that degrades mRNA of the TET2 gene, an antisense oligonucleotide that reduces TET2 protein expression, or the like.

In an exemplary embodiment, the TET2 inhibitor may be shRNA.

The "shRNA" is intended to overcome disadvantages of siRNA, such as expensive biosynthesis costs and short-term persistence of RNA interference effect due to low cell transfection efficiency, and may be expressed from a promoter of RNA polymerase III using an adenoviral, lentiviral, or plasmid expression vector system introduced into cells. The shRNA is known to induce silencing of a target gene after being converted into siRNA having an accurate structure by an siRNA processing enzyme (Dicer or Rnase III) existing in cells. The siRNA refers to a short double-stranded RNA capable of inducing RNAi phenomenon by cleaving a specific mRNA, and consists of a sense RNA strand having a sequence homologous to mRNA of a target gene and an antisense RNA strand having a sequence complementary thereto. Since the shRNA or siRNA may inhibit the expression of the target gene, the shRNA or siRNA may be provided as an efficient gene knockdown method or a method of gene therapy.

In an exemplary embodiment, the nucleic acid encoding shRNA may have a nucleotide sequence of SEQ ID NO: 10 or SEQ ID NO: 18, may be synthesized or modified shRNA such as a homologue, allotype, variant, derivative, or fragment thereof, and may be, for example, shRNA in which a loop sequence (an underline of Table 1) existing at the center in SEQ ID NO: 10 or SEQ ID NO: 18 is modified.

The TET2 inhibitor may have a nucleotide sequence represented by SEQ ID NO: 1 or SEQ ID NO: 9 as a target sequence.

The "target sequence" is a nucleotide sequence present in a target gene or nucleic acid, may be a nucleic acid sequence complementary to the nucleic acid sequence contained in the TET2 inhibitor, and may be, for example, a nucleic acid sequence that is at least 70%, 75%, 80%, 85%, 90%, or 95% or more complementary or completely complementary.

In an exemplary embodiment, the TET2 inhibitor may be shRNA that forms a complementary bond to a target sequence of SEQ ID NO: 1 or SEQ ID NO: 9 in a nucleic acid sequence of TET2.

In a specific embodiment of the present invention, as a result of introducing shRNA that binds to various target sequences contained in the TET2 gene and then confirming an expression level of the TET2 gene by Western blot, it is confirmed that shRNA that forms a complementary bond to the target sequence of SEQ ID NO: 1 or SEQ ID NO: 9 inhibits the expression of the TET2 gene significantly more than shRNA that binds to another target sequence.

The immune cells are cells involved in the immune response and include all cells directly or indirectly involved in the immune response and pre-differentiated cells thereof. The pre-differentiated cells may be embryonic stem cells, adult stem cells, or induced pluripotent stem cells that have self-replication and differentiation capabilities.

The immune cells may be immune cells derived from humans or immune cells derived from non-human mammals such as a dog, a cat, a pig, and a mouse. In addition, the immune cells may be obtained by isolating and purifying from immune cells infiltrating a body fluid such as blood or bone marrow fluid, tissue such as spleen, thymus, or lymph node, or cancer tissue such as a primary tumor, a metastatic tumor, or cancerous ascites.

The immune cells may be CD3 positive cells, for example, T cells.

The immune cells may be CD56 positive cells, for example, NK cells.

The immune cells may be CD3 and CD56 double positive cells, for example, natural killer T (NKT) cells or cytokine induced killer (CIK) cells.

Another aspect of the present invention provides a method for preparing immune cells with improved activity, the method including injecting a TET2 inhibitor into immune cells.

The descriptions of the immune cells and the TET2 inhibitor are the same as described above.

The injection may be performed in vivo or in vitro.

The injection method may be one or more methods selected from electroporation, a liposome, a plasmid, a viral vector, and nanoparticles.

The viral vector may be one or more selected from the group consisting of retrovirus, lentivirus, adenovirus, adeno-associated virus, vaccinia virus, poxvirus, and herpes simplex virus.

The TET2 inhibitor may be miRNA, siRNA, or shRNA that degrades mRNA of the TET2 gene, an antisense oligonucleotide that reduces the expression of the TET2 protein, or the like, and shRNA may be used in terms of economic efficiency and sustainability.

Still another aspect of the present invention provides a recombinant vector containing a nucleic acid encoding a TET2 inhibitor.

In a specific embodiment, the TET2 inhibitor may be shRNA.

A vector useful for transferring the shRNA may be a viral vector, and the virus may be one or more selected from the group consisting of retrovirus, lentivirus, adenovirus, adeno-associated virus, vaccinia virus, poxvirus, and herpes simplex virus.

The recombinant vector may be constructed by a recombinant DNA method known in the art.

In order to introduce the shRNA into the virus, a nucleic acid having a nucleotide sequence set forth in SEQ ID NO: 10 or SEQ ID NO: 18 may be prepared based on the shRNA sequence.

It is preferable that the shRNA that inhibits the expression of TET2 is operably linked to at least a promoter in order to be properly transcribed in the transferred cells. The promoter may be any promoter that may function in eukaryotic cells. For example, a U6 promoter, which is advantageous for producing small size RNA as RNA polymerase III, may be used. For efficient transcription of shRNA that inhibits TET2 expression, a regulatory sequence such as a leader sequence, a polyadenylation sequence, a promoter, an enhancer, an upstream activation sequence, a signal peptide sequence, or a transcription terminator may be additionally contained as needed.

The "operably linked" refers to a functional linkage between nucleic acid sequences. A case where arbitrary nucleic acid sequences are operably linked is a case where any nucleic acid sequence is positioned to be functionally related to another nucleic acid sequence. For example, a case where any transcriptional regulatory sequence affects transcription of shRNA means that the transcriptional regulatory sequence is operably linked to the shRNA.

The recombinant vector may additionally contain a nucleic acid encoding a chimeric antigen receptor (CAR).

The "nucleic acid encoding a CAR" is not particularly limited as long as it is a nucleic acid encoding a polypeptide constituting a CAR, and includes a nucleic acid encoding polypeptide of a single-chain antibody that recognizes a cell surface antigen of a cancer cell, a transmembrane domain, and an intracellular signaling domain.

The single-chain antibody consists of a light chain variable region and a heavy chain variable region (scFv) derived from an antigen binding site of a monoclonal antibody, and includes an oligo or polypeptide in which a linker peptide is located between the light chain variable region and the heavy chain variable region.

The cell surface antigen of the cancer cell recognized by the single-chain antibody may be a biomolecule specifically expressed in cancer cells and progenitor cells thereof, a biomolecule whose expression is newly confirmed due to cell cancerization, or a biomolecule whose expression level is increased in cancer cells compared to normal cells, and may be, for example, CD20, EGFR, FITC, CD19, CD22, CD33, PSMA, GD2, EGFR variant, ROR1, c-Met, HER2, CEA, mesothelin, GM2, CD7, CD10, CD30, CD34, CD38, CD41, CD44, CD74, CD123, CD133, CD171, MUC16, MUC1, CS1(CD319), IL-13 Ra2, BCMA, LewisY, IgG kappa chain, Folate receptor-alpha, PSCA, or EpCAM.

The intracellular signaling domain is a domain capable of transducing signals within the cell when the single chain antibody recognizes a cell surface antigen of a cancer cell, and may include at least one or two or more selected from polypeptides of an intracellular domain of CD28, 4-1BB (CD137), GITR, CD27, OX40, HVEM, CD3ζ, or Fc Receptor associated γ chain.

When two or more polypeptides of the intracellular domain are included, these polypeptides may be linked through an oligopeptide linker or polypeptide linker consisting of 2 to 10 amino acids, and such a linker sequence may be, for example, a glycine-serine continuous sequence.

The transmembrane domain may be a polypeptide of a transmembrane domain derived from CD8, α and β chains of a T cell receptor, CD28, CD3ε CD45, CD4, CD5, CD8, CD9, CD16, CD22, CD33, CD37, CD64, CD80, CD86, CD134, CD137, CD154, or GITR. The CAR is immobilized to a cell membrane of an immune cell such as a T cell by such a transmembrane domain.

A spacer region consisting of any oligopeptide or polypeptide may be installed between the single-chain antibody that recognizes the cell surface antigen of the cancer cell, and the transmembrane domain and the intracellular signaling domain. A length of the spacer region may be 1 to 100 amino acids or 10 to 50 amino acids, and such a spacer region may be, for example, a glycine-serine continuous sequence.

The nucleic acid encoding the CAR and the nucleic acid encoding the TET2 inhibitor may include any nucleic acid therebetween as long as each nucleic acid may be expressed, and may be linked, for example, through a sequence encoding a self-cleavable peptide (2A peptide) or an internal ribozyme entry site (IRES). Each nucleic acid may be efficiently expressed by linking the nucleic acids using such a sequence.

The nucleic acid encoding the CAR may be prepared by a known technique such as a chemical synthesis method based on a nucleotide sequence encoding polypeptides of a single-chain antibody against a cell surface antigen of a cancer cell, a transmembrane domain, and an intracellular signaling domain, or an amplification method by PCR.

In addition, information on the nucleotide sequence encoding the polypeptides of the single-chain antibody against a cell surface antigen of a cancer cell, the transmembrane domain, and the intracellular signaling domain may be appropriately obtained by searching known literature or databases such as NCBI (http://www.ncbi.nlm.nih.gov/guide/) .

Still another aspect of the present invention provides immune cells with improved activity into which the recombinant vector is introduced.

The activity improvement may be one or more selected from the group consisting of survival rate improvement, proliferation rate improvement, and cytokine expression increase.

The immune cells with improved activity may be immune cells in which TET2 is inactivated.

The inactivation of TET2 may be achieved through regulation of expression of the TET2 gene or protein, and since TET2 is an endogenous gene that affects proliferation, survival, and/or function of immune cells, the expression of the TET2 gene or protein may be regulated by targeting some or all of noncoding or coding regions of TET2.

The expression regulation of TET2 may be achieved through mRNA degradation or translation inhibition, and the regulation results may include both knockdown and knockout forms.

The "knockdown" means a reduction in transcription and/or translation of a target gene or nucleic acid or a reduction in expression of a target protein. The activity of the immune cells may be improved by regulating the expression of the over-expressed TET2 gene or protein through knockdown.

The "knockout" means inactivation of a target gene or nucleic acid, and the inactivation of the target gene or nucleic acid means a state in which transcription and/or translation of the target gene or nucleic acid is not possible. The transcription and translation of the TET2 gene may be inhibited through knockout, and thus, the expression of the TET2 protein may be prevented.

The targeting may be performed, for example, to target a promoter region or a transcription sequence of the TET2 gene, such as an intron or exon sequence, and a coding sequence, for example, a coding region or an early coding region may be a target for alternation or knockout of expression.

In a specific embodiment, in the expression regulation of the gene or protein, after shRNA is injected into the cell, an RNAi mechanism occurs to degrade mRNA of the TET2 gene or inhibit translation, such that the TET2 gene may be inactivated.

The immune cells are cells involved in the immune response and include all cells directly or indirectly involved in the immune response and pre-differentiated cells thereof. The pre-differentiated cells may be embryonic stem cells, adult stem cells, or induced pluripotent stem cells that have self-replication and differentiation capabilities.

The immune cells may be immune cells derived from humans or immune cells derived from non-human mammals such as a dog, a cat, a pig, and a mouse. In addition, the immune cells may be obtained by isolating and purifying from immune cells infiltrating a body fluid such as blood or bone marrow fluid, tissue such as spleen, thymus, or lymph node, or cancer tissue such as a primary tumor, a metastatic tumor, or cancerous ascites.

The immune cells may be CD3 positive cells, for example, T cells.

The immune cells may be CD56 positive cells, for example, NK cells.

The immune cells may be CD3 and CD56 double positive cells, for example, natural killer T (NKT) cells or cytokine induced killer (CIK) cells.

The T cells may be one or more selected from the group consisting of αβT cells, γδT cells, CD8 positive T cells (e.g., CD8+ naive T cells, CD8+ effector T cells, central memory T cells, or effector memory T cells), CD4 positive T cells, natural killer T (NKT) cells, regulatory T cells (Treg), memory T cells, tumor-infiltrating T cells, lymphoid progenitor cells, hematopoietic stem cells, and T cells isolated from peripheral blood mononuclear cells (PBMCs). In a specific embodiment, the immune cells may be immune cells that express a chimeric antigen receptor (CAR). The single-chain antibody expressed by the CAR-expressing immune cell is located in the extracellular region, and such a single-chain antibody is provided, such that the CAR-expressing immune cell may recognize a tumor-associated antigen (TAA) expressed on the surface of the cancer cell.

In a specific embodiment, the chimeric antigen receptor (CAR) may contain an antibody that specifically binds to CD19 or an antigen-binding fragment thereof. The CD19 is a cluster designation (cluster of differentiation (CD)) assigned the number 19, which identifies cell surface molecules according to immunophenotype, and it is known that the CD19 is expressed on most B cells and malignant cancer cells and provides an ideal target for these carcinomas.

A method for introducing the recombinant vector into immune cells is not particularly limited, and examples thereof include known methods such as a viral infection method, a calcium phosphate method, a lipofection method, microinjection, and electroporation.

The viral infection method may be a method of transfecting the recombinant vector and a packaging plasmid into packaging cells to produce a recombinant virus, and infecting T cells with the recombinant virus.

The introduction of the recombinant vector into immune cells may be confirmed by examining the expression of the CAR by PCR such as flow cytometry, Northern blotting, Southern blotting, or RT-PCR, ELISA, or Western blotting.

Still another object of the present invention provides a pharmaceutical composition for treating cancer containing the immune cells with improved activity as an active ingredient.

In a specific embodiment of the present invention, it was confirmed that, when immunodeficient mice were treated with immune cells into which the TET2 inhibitor was introduced, the ability to suppress tumor growth was significantly improved and the survival rate of the immunodeficient mice was significantly increased compared to immune cells into which the TET2 inhibitor was not introduced.

The "cancer" and "tumor" are used interchangeably, and typically refer to or mean a physiological condition of a mammal characterized by unregulated cell growth/proliferation.

The cancer may include a vascularized tumor as well as a tumor that is not vascularized or is not yet substantially vascularized. The cancer may include a non-solid tumor (for example, hematologic tumors such as leukemia and lymphoma), or may include a solid tumor. Examples of the types of the cancer include, but are not limited to, carcinoma, blastoma, sarcoma, and specific leukemic or lymphoid malignancy, and benign and malignant tumors, for example, sarcoma, carcinoma, and melanoma. An adult tumor/cancer and a pediatric tumor/cancer are also included.

The hematologic cancer is cancer of the blood or bone marrow. Examples of the hematological (or hematogenous) cancer include leukemias, including acute leukemias (for example, acute lymphocytic leukemia, acute myelocytic leukemia, myeloblastic, prolymphocytic, myelomonocytic, monocytic, and erythroleukemia), chronic leukemias (for example, chronic lymphocytic (granulocytic) leukemia, chronic myelocytic leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia, and myelodysplasia.

The solid tumor is an abnormal mass of tissue that usually does not contain cysts or liquid areas. The solid tumor may be benign or malignant. Different types of solid tumors are named for the type of cells that form them (for example, sarcoma, carcinoma, and lymphoma). Examples of the solid tumors such as sarcoma and carcinoma include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, colorectal cancer, gastric cancer, pancreatic cancer, breast cancer, lung cancer, ovarian cancer, prostate cancer, laryngopharyngeal cancer, hepatocellular carcinoma, squamous cell carcinoma, basal cell carcinoma, adenocarcinoma, sweat gland carcinoma, medullary thyroid carcinoma, papillary thyroid carcinoma, pheochromocytoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (for example, glioma (for example, brainstem glioma and mixed gliomas), glioblastoma (also known as glioblastoma multiforme), astrocytoma, CNS lymphoma, germinoma, medulloblastoma, Schwannoma craniopharyogioma, ependymoma, pinealoma, hemangioblastoma, acoustic neuroma, oligodendroglioma, meningioma, neuroblastoma, retinoblastoma, and brain metastases).

The "treatment" means all actions that inhibit cancer or delay progression of cancer by administering the composition of the present invention, and includes inhibition of development of cancer and alleviation or elimination of symptoms.

The pharmaceutical composition may additionally contain a pharmaceutically acceptable excipient. Examples of the excipient include a surfactant, and preferably a polysorbate-based non-ionic surfactant; a buffer such as neutral buffered saline or phosphate-buffered saline; sugars or sugar alcohols such as glucose, mannose, sucrose or dextran, and mannitol; an amino acid such as glycine or histidine or a protein or polypeptide; an antioxidant; a chelating agent such as EDTA or glutathione; a penetrant; an adjuvant; and a preservative, but are not limited thereto.

The pharmaceutical composition may be formulated by using a method known in the art so as to provide rapid, sustained, or delayed release of active ingredients after being administrated to mammals except for the human. The formulation may be powders, granules, tablets, emulsions, syrups, aerosols, soft or hard gelatin capsules, sterile injectable solutions, and sterile powder forms.

### MODE FOR CARRYING OUT THE INVENTION

Hereinafter, the present invention will be described in detail with reference to Examples and Experimental Examples. However, the following Examples and Experimental Examples illustrate only the present invention in detail, and the present invention is not limited by the following Examples and Experimental Examples.

### [Example 1]

### Selection of Target and shRNA that Effectively Induce Silencing of Tet2

In order to find a target sequence that effectively induces silencing of Tet2, shRNA binding to various target sequences and viral vectors expressing shRNA were prepared and infected into 293T cells, and then, only gene-transduced cells were obtained through Puromycin selection, and western blot was performed using Tet2 antibody.

### <1-1>. Construction of Tet2 Knockdown Plasmid

In order to knock down Tet2, the shRNAs listed in Table 1 were each cloned into pLKO.1-puro vector. The pLKO.1-puro vector (#8453) purchased from Addgene was cleaved with AgeI and EcoRI. Sense primers and antisense primers having the target sequences in Table 1 were custom-made by Macrogen. The sense primers and antisense primers were annealed. A primer double strand was cloned into pLKO.1-puro vector using lagase.

**[Table 1]**

| **shRNA No.** | **Target sequence of shRNA** | SEQ ID NO : | **Nucleic acid encoding Tet2 shRNA** | SEQ ID NO : |
|---|---|---|---|---|
| A | TTTCACGCCAAGTCGTTATT T | 1 | CCGG TTTCACGCCAAGTCGTTATTTGCTAGC AAATAACGACTTGGCGTGAAA TTTTTG | 10 |
| B | GCACACACATGTATATGTAT A | 2 | CCGG GCACACACATGTATATGTATAGCTAGC TATACATATACATGTGTGTGC TTTTTG | 11 |
| C | GCACCACCAGAAAACAAAA | 3 | CCGG GCACCACCAGAAAACAAAAGCTAGC TTTTGTTTTCTGGTGGTGC TTTTTG | 12 |
| D | GGATGACCCAAAAGAGGAA | 4 | CCGG GGATGACCCAAAAGAGGAAGCTAGC TTCCTCTTTTGGGTCATCC TTTTTG | 13 |
| E | GGAAACTAGAAGCCAAGAA | 5 | CCGG GGAAACTAGAAGCCAAGAAGCTAGC TTCTTGGCTTCTAGTTTCC TTTTTG | 14 |
| F | GCAATCCAAACATGGACTA | 6 | CCGG GCAATCCAAACATGGACTAGCTAGC TAGTCCATGTTTGGATTGC TTTTTG | 15 |
| G | GCATCTAGGAAAAGACCAA | 7 | CCGG GCATCTAGGAAAAGACCAAGCTAGCTTGGTCT TTTCCTAGATGC TTTTTG | 16 |
| H | GCATAGTCATGGAACACAA | 8 | CCGG GCATAGTCATGGAACACAAGCTAGCTTGTGTT CCATGACTATGC TTTTTG | 17 |
| I | GAAGCAGTTTCACGCCAAGT C | 9 | CCGG GAAGCAGTTTCACGCCAAGTCGCTAGCGACTT GGCGTGAAACTGCTTC TTTTTG | 18 |

### <1-2>. Production of Lentivirus

293T cells were cultured in DMEM medium (SH30243.01, Hyclone) containing 10% FBS (16000-044, Hyclone). A transfer vector (pLKO.1-puro vector), a packaging vector, and an envelope vector were co-transfected into the 293T cells. After 24 hours, the medium was changed and virus soup was collected after 24 and 48 hours. The virus soup was filtered with a 0.45 um filter (SLHPR33TB, Millipore corp.) and stored at -70°C.

### <1-3>. Preparation of 293T Cells In Which Tet2 Is Inactivated

The pLKO.1 virus soup obtained in Example 1-2 was infected into 293T cells using polybrene (H9268, Sigma). After 3 days, the cells were treated with puromycin (1 µg/ml). The cells were continued to grow for longer than a week, and only 293T cells containing the gene were selected.

After preparing a cell lysate, western blot was performed using Tet2 antibody (18950, CST).

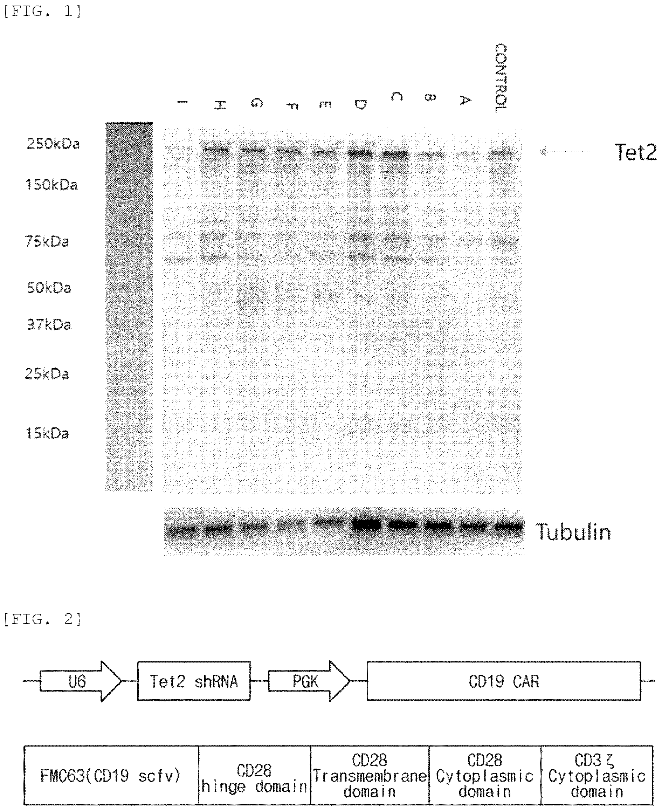

As a result, as illustrated in FIG. 1, it was confirmed that among nine shRNAs, sh-A and sh-I effectively inhibited the expression of Tet2. Through this, a target sequence and shRNA effectively silencing tet2 were selected.

### [Example 2]

### Preparation of CAR-T Cells In Which Tet2 Is Inactivated

CAR-T cells were prepared using sh-A and sh-I which effectively knocked down Tet2 in 293T cells.

First, pLKO.1-tet2 sh-A and pLKO.1-tet2 sh-I vectors in which shRNA was cloned were cleaved with BamHI and NsiI. CD19 CAR gene was subjected to PCR and then inserted into pLKO.1 sh-A and pLKO.1-tet2 sh-I vectors using ligase to obtain vectors that simultaneously implement Tet2 knockdown and CD19-CAR expression (FIG. 2). Using each vector, lentiviral particles were prepared through 293T cells.

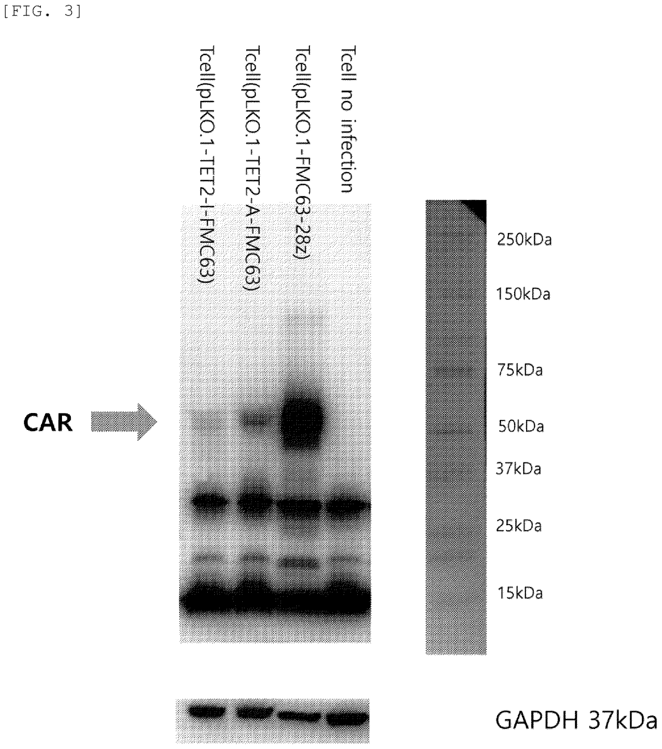

Next, PBMCs were proliferated in a 100 mm culture plate. Dynabead human T-activator CD3/CD28 (11132D, Thermofisher) and IL-2 (202-IL-500, R&D system) were added thereto. 24 hours later, T cells were infected with lentivirus containing pLKO.1 sh-A vector and pLKO.1-tet2 sh-I vector. 4 days after infection, the expression of the CAR was confirmed through western blot using a lysate (FIG. 3). To this end, CD3ζ antibody (51-6527GR, BD bioscience) was used.

### [Example 3]

### Effect of Reducing Expression of Immunosuppressive Receptor by TET2 Inactivation

An immunosuppressive receptor is known to function to suppress the activation of T cells. In order to confirm the effect of TET2 inactivation on the activation of immune cells, changes in the expression of immunosuppressive receptors in T cells, CAR-T cells, and CAR-T cells in which TET2 was inactivated were measured through FACS analysis.

Specifically, T cells, CD19 CAR T cells, and TET2-shRNA-CD19 CAR T cells were prepared at 3 × 10⁶ cells, respectively, and the samples were each washed twice with 1 mL of a cold stain buffer (0.2% BSA, 0.08% NaN3 in PBS). (4°C, 4,000 rpm, 5 min) The supernatant from the last washing step was discarded, and each sample was dissolved in 300 µL of the cold stain buffer. The dissolved sample was divided into three, 3 µL of each of Pacific Blue^{™} anti-human CD8 Antibody (344718, biolegend) and PE anti-human CD279 (PD-1) Antibody (329905, biolegend) was added to one sample, 3 µL of each of Pacific Blue^{™} anti-human CD8 Antibody (344718, biolegend) and Human TIM-3 PE-conjugated Antibody (Fab2365P, R&D systems) was added to the other one, and no antibody was added to the remaining one. Thereafter, the sample was reacted in a light-blocked environment at 4°C for 20 minutes, and the sample was washed twice with 1 mL of the cold stain buffer. (4°C, 4,000 rpm, 5 min) The supernatant from the last washing step was discarded, the cells were dissolved in 250 µL of Fixation buffer (4% Formaldehyde in PBS), and the cells were reacted in an environment at 4°C without light for 10 minutes. After completing the reaction, the sample was washed twice with 1 ml of the cold stain buffer. (4°C, 4,000 rpm, 5 min) Then, the supernatant from the last washing step was discarded, and the cells of each sample were dissolved in 500 µL of the cold stain buffer. After O/N, the cells were separated using a cell strainer snap cap tube, and fluorescence was measured using a FACS machine.

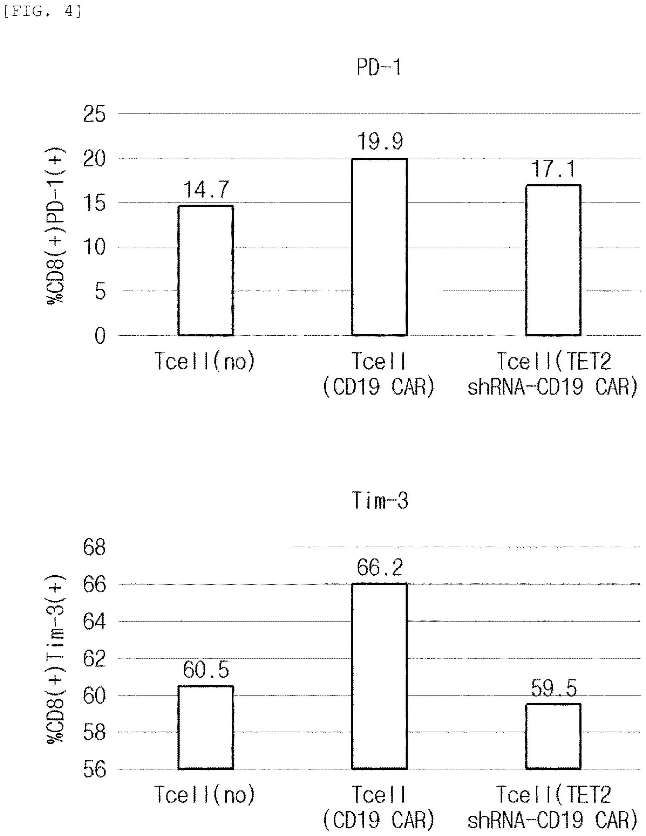

As a result, as illustrated in FIG. 4, it was shown that the expression levels of both PD-1 and Tim-3 increased in the CAR-T cells compared to normal T cells, but the expressions decreased again in the CAR-T cells in which TET2 was inactivated.

It was confirmed from this that the activity of the CAR-T cells in which TET2 was inhibited by shRNA was improved compared to the existing CAR-T cells.

### [Example 4]

### Improved In Vivo Anticancer Efficacy of CAR-T Cells In Which Tet2 Is Inactivated

In order to confirm the in vivo anticancer efficacy of the CAR-T cells prepared in Example 2, the CAR-T cells were administered to immunodeficient mice, and then tumor proliferation in vivo and mouse survival rates were measured.

Specifically, immunodeficient NSG mice (Charles River) were injected intravenously through the tail (IV injection) with 1 × 10⁶ CD19 positive NALM6-luc cells, which are tumor cells expressing luciferase. The next day, 1 × 10⁷ CAR-T cells prepared in Example 3 were injected intravenously. Thereafter, 100 µL of 30 mg/mL Luciferin (122799, PerkinElmer) was injected intraperitoneally (IP injection), and then, after one week, anticancer activity was observed by checking the intensity of in vivo luminescence emitted by the tumor cell population using an imaging device (IVIS, PerkinElmer).

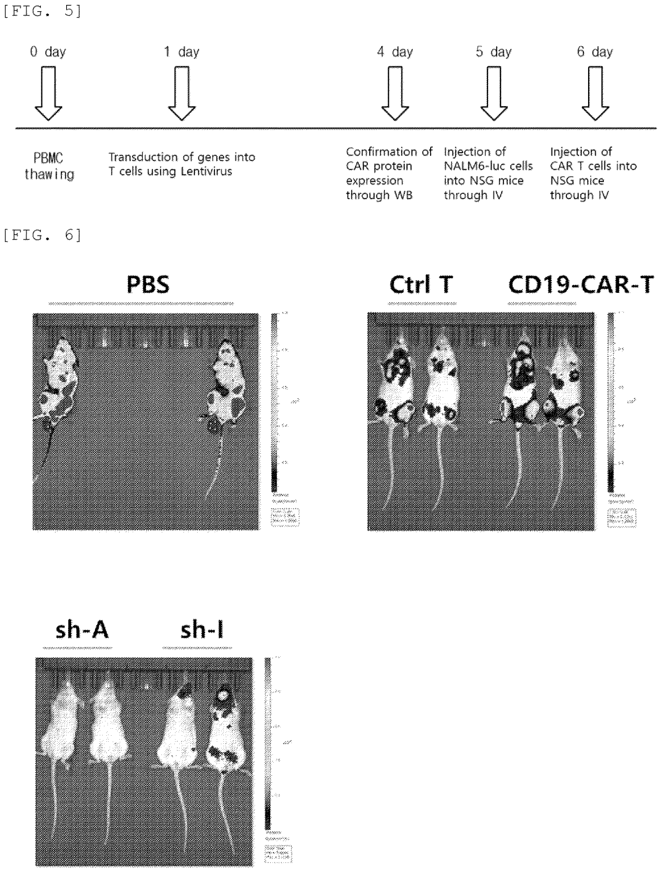

As a result, as illustrated in FIG. 6, it was observed that when the CD19 CAR-T cells expressing sh-A or sh-I were injected, tumor growth was significantly inhibited compared to when the existing CD19 CAR-T cells were administered. In particular, it was confirmed that all tumor cells were removed in the group to which the CD19 CAR-T cells expressing sh-A were administered.

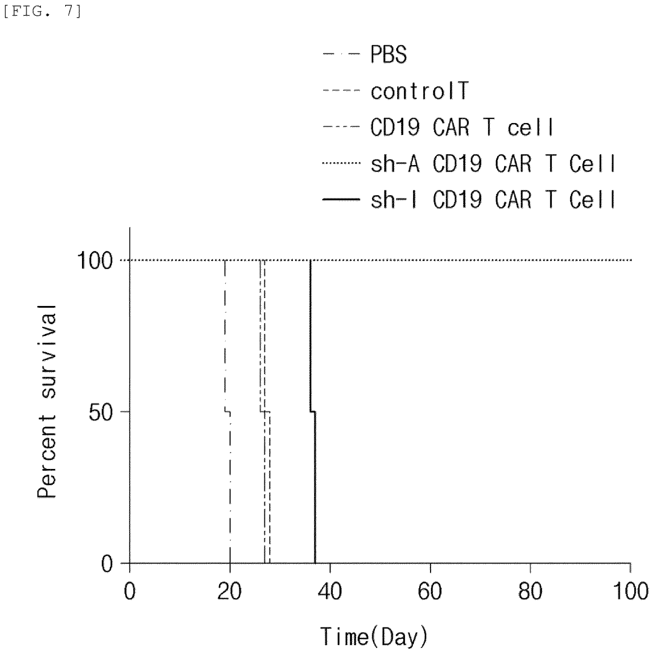

As a result, in the group to which the existing CD19 CAR-T cells were administered, all individuals died within 30 days after tumor inoculation, but the mice to which the CAR-T cells expressing sh-I and the CAR-T cells expressing sh-A were administered survived even after 30 days, and in particular, in the case of the group to which the CAR-T cells expressing sh-A were administered, all individuals survived until 80^{th} day when the experiment was completed (FIG. 7).

It was confirmed from this that the CAR-T cells in which TET2 was suppressed by shRNA, particularly, the CAR-T cells expressing sh-A, showed significantly improved therapeutic efficacy compared to the existing CAR-T cells.

### [Example 5]

### Comparison of Tumor Cell Lytic Activity of CD19 CAR T Cells Containing sh-A or sh-I

In order to confirm that in vitro anticancer efficacy of the CD19 CAR-T cells expressing sh-A or sh-I prepared in Example 2, after the CAR-T cells were administered to cells expressing CD19 or cells not expressing CD19, tumor cell suppressive activity was compared as follows.

Specifically, control T cells, CD19 CAR T cells targeting CD19, and the CD19 CAR-Tet2 sh A cells and CD19 CAR-Tet2 sh I cells prepared in Example 2 were co-cultured with NALM6 cells expressing CD19 or 293T cells not expressing CD19 in a 96-well plate at an E:T ratio of 10:1. After 4 hours of culture, lytic properties of the target cells were confirmed by measuring luminescence using luciferase expressed in NALM6 cells or 293T cells not expressing CD19.

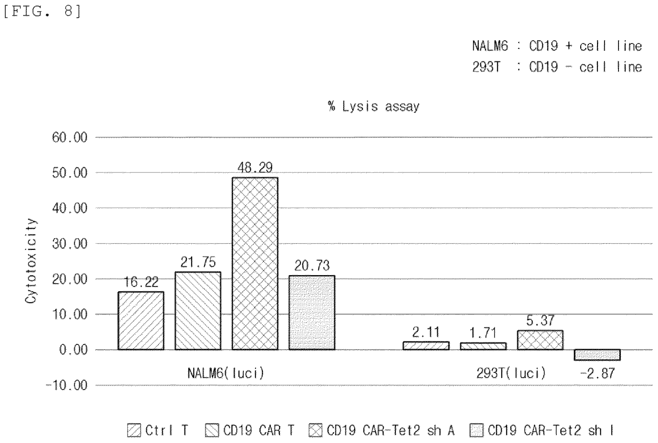

As a result, as illustrated in FIG. 8, it was confirmed that the CD19 CAR-T cells expressing sh-A showed lytic properties that killed about 50% of NALM6 tumor cells expressing CD19, but in contrast, the CD19 CAR-T cells expressing sh-I showed a similar level of tumor cell lytic properties as the CD19 CAR T cells that do not inhibit TET2. Furthermore, it was confirmed that all experimental groups showed almost no cell lytic properties to the 293T cells not expressing CD19.

It can be seen from this that the CD19 CAR-T cells expressing sh-A increase the activity of the CAR T cells targeting CD19 and exhibit significantly excellent anti-tumor effects.

## Claims

1. A composition for improvement of immune cell activity,
comprising a tet methylcytosine dioxygenase 2 (TET2) inhibitor,
wherein the TET2 inhibitor forms a complementary bond to a target sequence of SEQ ID NO: 1 or SEQ ID NO: 9 in a nucleic acid sequence of TET2.

2. The composition of claim 1, wherein the improvement of immune cell activity is one or more selected from the group consisting of survival rate improvement, proliferation rate improvement, and cytokine expression increase.

3. The composition of claim 1, wherein the TET2 inhibitor comprises miRNA, siRNA, shRNA, or an antisense oligonucleotide.

4. The composition of claim 3, wherein a nucleic acid encoding the shRNA has a nucleotide sequence of SEQ ID NO: 10 or SEQ ID NO: 18.

5. The composition of claim 1, wherein the immune cells are immune cells expressing a chimeric antigen receptor (CAR).

6. The composition of claim 1, wherein the immune cells are T cells or NK cells.

7. A method for preparing immune cells with improved activity, the method comprising injecting a TET2 inhibitor into immune cells,
wherein the TET2 inhibitor forms a complementary bond to a target sequence of SEQ ID NO: 1 or SEQ ID NO: 9 in a nucleic acid sequence of TET2.

8. The method of claim 7, wherein the injection is performed by one or more methods selected from the group consisting of electroporation, a liposome, a plasmid, a viral vector, and nanoparticles.

9. A recombinant vector comprising a nucleic acid encoding a TET2 inhibitor.

10. The recombinant vector of claim 9, wherein the recombinant vector is one or more selected from the group consisting of retrovirus, lentivirus, adenovirus, adeno-associated virus, vaccinia virus, poxvirus, and herpes simplex virus.

11. The recombinant vector of claim 9, further comprising a nucleic acid encoding a chimeric antigen receptor (CAR).

12. The recombinant vector of claim 9, wherein the nucleic acid encoding the TET2 inhibitor has a nucleotide sequence of SEQ ID NO: 10 or SEQ ID NO: 18.

13. Immune cells with improved activity into which the recombinant vector of claim 9 is introduced.

14. The immune cells of claim 13, wherein the immune cells additionally contain a chimeric antigen receptor (CAR).

15. The immune cells of claim 14, wherein the chimeric antigen receptor contains an antibody that specifically binds to CD19 or an antigen-binding fragment thereof.

16. The immune cells of claim 13, wherein the immune cells are T cells or NK cells.

17. The immune cells of claim 16, wherein the T cells are helper T cells, cytotoxic T cells, memory T cells, regulatory T cells, or natural killer T cells.

18. The immune cells of claim 13, wherein the activity improvement is one or more selected from the group consisting of survival rate improvement, proliferation rate improvement, and cytokine expression increase.

19. A pharmaceutical composition for treating cancer, comprising the immune cells of any one of claims 13 to 18 as an active ingredient.
